# EUROPEAN PATENT APPLICATION

(11) **EP 0 978 239 A1**
(43) Date of publication of application: **09.02.2000**
(21) Application number: 98306267.0
(22) Date of filing: 05.08.1998
(51) Int. Cl.: A43D 1/02, A61B 5/107

(54) **Foot growth gauge**

(71) Applicant: Malthouse Hunter Limited, Sturminster Newton, Dorset DT10 2AU (GB)
(72) Inventor: Snook, Laurence Arthur, Sturminster Newton, Dorset DT10 2AU (GB)
(74) Representative: Craske, Stephen Allan

(57) **Abstract**

A body moulding 1 carries a separate foot support platform 5 and an integral heel stop 6 upstanding at the rear of the platform which is supported by an integral leg 8. A slider 12 is engaged with the platform 5 for sliding movement in a front-rear direction) and includes an upstanding toe stop 14. The platform 5 carries a length scale 21 from which the length of a foot can be measured according to the position of the slider along the platform, and a tape 20 is received in slots 19 on opposite sides of the slider 12 to form a width gauge for measuring the absolute width of the foot. The width gauge 20 is adjustable in a front-rear direction independently of the slider to allow the foot to be measured at its widest part. A relative width gauge is formed by a relative width scale 24, carried with the slider, and an absolute width scale 23, carried by the platform, comprises a series of parallel bands representing values of absolute width and which are inclined relative to the movement of the slider to intersect the relative width scale 24. Thus, the absolute width of the foot as measured by the absolute width gauge 20 can be read on the scale 23, and by noting the point at which the relevant band intersects the scale 24 the absolute width can be related to the length of the foot to provide a relative width reading.

## Description

### TECHNICAL FIELD OF THE INVENTION

This invention relates to foot growth gauges as used for measuring foot sizes for the purpose of fitting shoes. The subject gauge is particularly intended for use in measuring infants and childrens feet, although a version could be used for measuring adults feet.

### BACKGROUND

The present invention seeks to provide a new and inventive form of foot growth gauge.

### SUMMARY OF THE INVENTION

The present invention proposes a foot growth gauge comprising:
- a body which includes a foot support platform and a heel stop upstanding at the rear of the platform;
- a slider which is engaged with the body for sliding movement along the said platform in a front-rear direction and which includes a toe stop upstanding from the platform;
- a slider scale from which the length of a foot can be measured according to the position of the slider along the platform;
- a width gauge for measuring the absolute width of a foot placed on the platform, said width gauge being adjustable in a front-rear direction independently of the slider to allow the foot to be measured at its widest part; and
- a relative width gauge comprising a relative width scale, carried with the slider, which extends substantially normal to the movement of the slider, and an absolute width scale, fixed with the body, which comprises a series of parallel bands representing values of absolute width and which are inclined relative to the movement of the slider to intersect said relative width scale whereby the absolute width of the foot as measured by the absolute width gauge can be related to the length of the foot to provide a relative width reading.

It will be appreciated that the absolute values of length and width could be in recognised units such as units of shoe size or centimetres or example, or they could be in purely arbitrary units.

The width gauge may comprise a flexible element such as a tape. The flexible element is preferably slidably inserted through slots which extend in a front-rear direction through guide elements fixed with the slider on opposite sides of the platform.

The platform is preferably separate from the body moulding, and is preferably snap-engaged with the body moulding for ease of assembly. The heel stop is preferably integral with the body moulding. The body moulding preferably comprises an integral support member which projects downwardly from its rear end. The support member preferably comprises a downwardly projecting leg and, at the lower end of the leg, a forwardly projecting foot.

### BRIEF DESCRIPTION OF THE DRAWINGS

The following description and the accompanying drawings referred to therein are included by way of non-limiting example in order to illustrate how the invention may be put into practice. In the drawings:
Figure 1 is a plan view of a foot growth gauge in accordance with the invention,
Figure 2 is a side view of the gauge,
Figure 3 is a front view of the gauge, looking from the right in Fig.s 1 and 2,
Figure 4 is a longitudinal vertical section through the gauge,
Figure 5 is staggered transverse section V-V of Fig. 1, and
Figure 6 is a detailed plan view of the platform and slider of the foot growth gauge showing the relative and absolute width gauge scales.

### DETAILED DESCRIPTION OF THE DRAWINGS

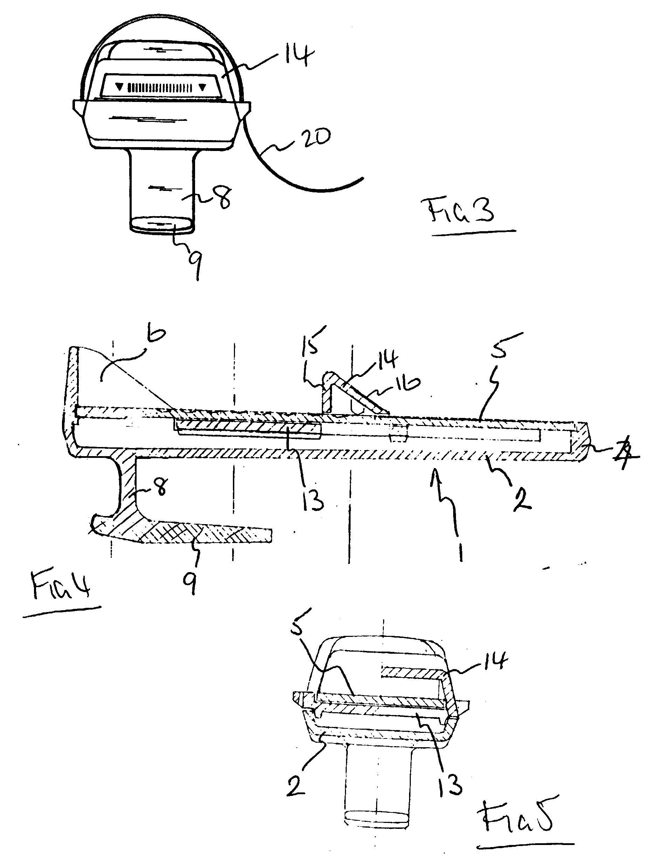

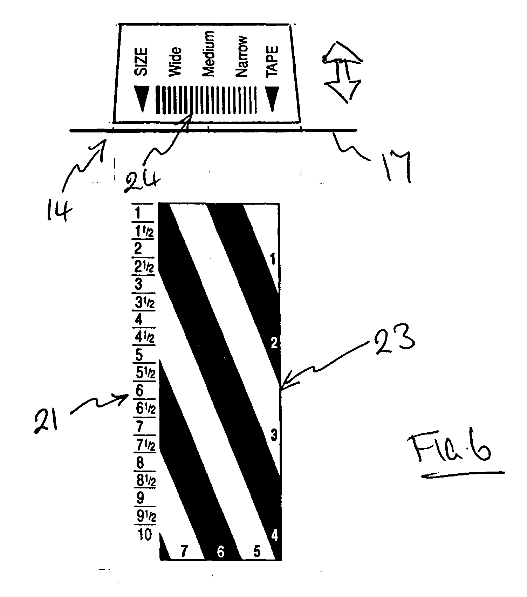

The foot growth gauge includes a flat, elongate moulding 1 including a base wall 2 (Fig. 4) having a curved rear end 3 and surrounded by an upstanding side wall 4. The moulding 1 carries a separate foot-support platform 5, also moulded of plastics, which is snap-engaged with the wall 4 at the front and rear end of the moulding for ease of assembly. At the rear end of the moulding the curved portion of the wall 4 is extended upwardly to act as a heel stop 6 when a foot is placed on the platform 5. Beneath the heel stop 6 the moulding includes an integrally formed depending leg 8 which ends in an integral, forwardly projecting support foot 9 to support the rear of the gauge in use. The leg 8 and foot 9 could be provided as a separate moulding which is secured to the base wall 2 using self-tapping screws, but although this makes the moulding easier the use of a separate moulding may result in an increase in the size of the gauge and increases assembly time and manufacturing cost.

The upper edge of the wall 3 has opposed shallow notches or recesses 11 on opposite sides of the platform 5 so that a slider 12 can be engaged with the platform for sliding movement along the platform in a front-rear direction. The slider 12 includes a shallow U-shaped wall 13 extending beneath the platform 5 (Fig. 4) which is bridged above the platform by an integral toe stop 14. It will be noted that the toe stop 14 includes a rear wall 15 which extends perpendicularly from the platform 5, and a front wall 16 which extends downwardly in a forward direction to meet the platform 2 along its front-most edge 17.

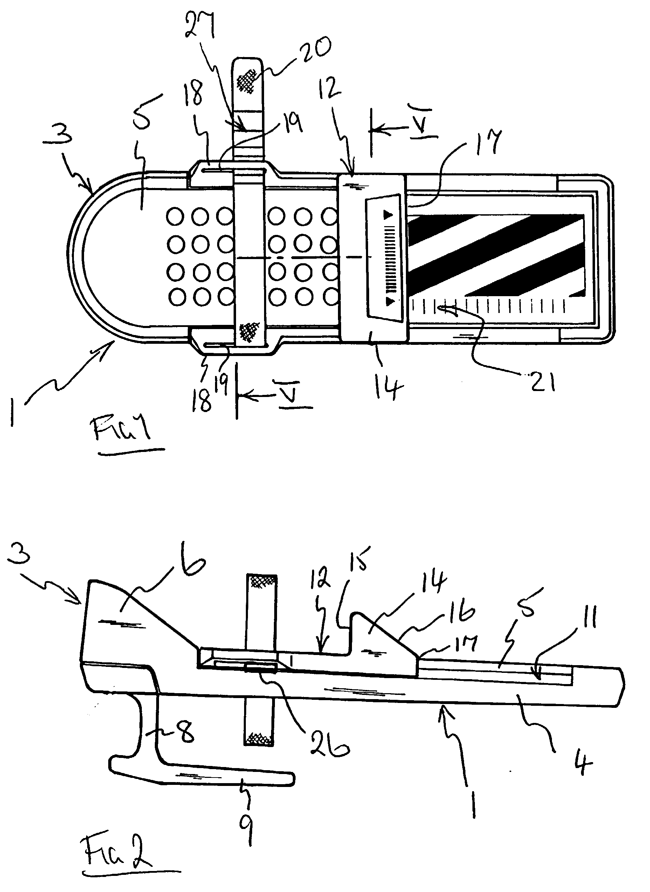

The wall 13 of the slider is extended on opposite sides to form a pair of narrow wings 18 (Fig. 1). The wings include slots 19 extending in a front-rear direction, through which a measuring tape 20 is inserted. One end of the tape is prevented from being pulled out of the slot 19 by a head 26 (Fig. 2) secured to the tape. When a foot is placed on the platform the heel is placed against the heel stop 6 and the slider 12 is moved along the platform until the rear wall 15 of the toe stop 14 just touches the toes. The measuring tape is slid along the slots 19 to the widest part of the foot, and the tape is then pulled around the foot allowing the absolute width of the foot to be read off against the edge of the slot 19 using a width scale 27 which is marked on the tape in suitable units of measurement.

With the slider in the same position, the front edge 17 of the toe stop 14 is used to read off the absolute length of the foot using a length scale 21 (Fig.s 1 and 6) marked on the upper surface of the platform, as shown in Fig. 6. The length may conveniently be indicated in shoe sizes.

It will also be noted that the platform also carries a second scale 23 which is inclined relative to the direction of movement of the slider 14, and in addition, a relative width scale 24 is carried on the inclined front wall 16 of the toe stop 14. The second scale 23 comprises a series of mutually parallel bands corresponding to the absolute width readings obtained from the measuring tape 20. These two scales are used to ascertain the correct width fitting as follows. The band corresponding to the absolute value of the width measurement obtained using the tape 20 is located on the second scale 23, and the point at which this band intersects the edge 17 of the slider is noted. The relative width can then be ascertained from the scale 24 which indicates whether the foot requires a wide, medium or narrow shoe fitting.

It will be noted that for any given band corresponding to a certain measured foot width, the relative width will change from wide to medium to narrow as the slider moves forwards along the platform. This feature automatically takes account of the fact that a short foot with a given width may be relatively wide whereas a longer foot with the same width will be relatively narrow.

The gauge is inexpensive to manufacture and easy to use, enabling parents to obtain an accurate measurement of a childs absolute foot size as well as relative width.

It will be appreciated that the features disclosed herein may be present in any feasible combination. Whilst the above description lays emphasis on those areas which, in combination, are believed to be new, protection is claimed for any inventive combination of the features disclosed herein.

## Claims

1. A foot gauge characterised by:
- a body which includes a foot support platform and a heel stop upstanding at the rear of the platform;
- a slider which is engaged with the body for sliding movement along the said platform in a front-rear direction and which includes a toe stop upstanding from the platform;
- a slider scale from which the length of a foot can be measured according to the position of the slider along the platform;
- a width gauge for measuring the absolute width of a foot placed on the platform, said width gauge being adjustable in a front-rear direction independently of the slider to allow the foot to be measured at its widest part; and
- a relative width gauge comprising a relative width scale, carried with the slider, which extends substantially normal to the movement of the slider, and an absolute width scale, fixed with the body, which comprises a series of parallel bands representing values of absolute width and which are inclined relative to the movement of the slider to intersect said relative width scale whereby the absolute width of the foot as measured by the absolute width gauge can be related to the length of the foot to provide a relative width reading.

2. A foot gauge according to Claim 1, in which the width gauge comprises a flexible element.

3. A foot gauge according to Claim 2, in which the flexible element is slidably inserted through slots which extend in a front-rear direction through guide elements fixed with the slider on opposite sides of the platform.

4. A foot gauge according to any preceding claim, in which the platform is separate from the body moulding.

5. A foot gauge according to Claim 4, in which the platform is snap-engaged with the body moulding for ease of assembly.

6. A foot gauge according to any preceding claim, in which the heel stop is integral with the body moulding.

7. A foot gauge according to any preceding claim, in which the body moulding comprises an integral support member which projects downwardly from the rear end of the body moulding.

8. A foot gauge according to Claim 7, in which the support member comprises a downwardly projecting leg and, at the lower end of the leg, a forwardly projecting foot.
